# EUROPEAN PATENT APPLICATION

(11) **EP 1 532 947 A1**
(43) Date of publication of application: **25.05.2005**
(21) Application number: 03425713.9
(22) Date of filing: 31.10.2003
(51) Int. Cl.: A61F 2/42

(54) **Articulated joint for orthopaedic use**

(71) Applicant: ORTHOFIX INTERNATIONAL B.V., 1071 Amsterdam (NL)
(72) Inventor: Corradi, Maurizio, 43100 Parma (IT); Rossi, Luigi, 37019 Peschiera del Garda (Verona) (IT); Venturini, Daniele, 37064 Povegliano Veronese (Verona) (IT)
(74) Representative: Botti, Mario

(57) **Abstract**

An articulated joint (10) for orthopaedic use, which advantageously allows also predetermined lateral rotations, comprises a first half part (12), equipped with a fork-shaped end (13) with two wings (13a), and a second half part (14), equipped with a male end (15) which can be inserted in the fork (13), said ends (13, 15) being hinged to each other by means of a pin (16) and thus angularly movable around said pin (16), at least one ring-shaped insert (20) being interposed between facing walls of said fork (13) and of said male end (15), said at least one insert (20) being assembled around said pin (16), said insert (20) being equipped with a convex surface (24), of predetermined bending, facing said wing (13a) of the fork (13).

## Description

### Field of application

The present invention relates in its more general aspect to an articulated joint for orthopaedic use.

In particular, this invention relates to an articulated joint comprising a first half part, equipped with a fork-shaped end, and a second half part, equipped with a male end which can be inserted in the fork, said ends being hinged to each other.

A typical example of orthopaedic use of such articulated joints is the use in articular prosthesis and i.e. in mechanical devices suitable for recovering the functionality of an injured articulation.

Reference is made in particular to the non limiting example of an articular prosthesis reconstructing an articulation between the metacarpus and a phalanx or between two phalanxes of an hand or a foot. In this case, the two joint half parts are associated to the two respective bones to be reconnected, the hinging of the two articulated joint ends recovering the articulation functionality.

### Prior art

To reconstruct the articulation between the metacarpus and a phalanx or between two phalanxes, known articulated joint comprises a first half part, equipped with a two-wing fork-shaped end, and a second half part, equipped with a male end which can be inserted in the fork, said ends being hinged to each other by means of a pin. Therefore, the two bones being connected reacquire the ability of moving angularly, reciprocally, substantially around the pin axis of symmetry.

It is evident that the service life of the articulated joint must be as long as possible, therefore at least one ring-shaped insert such as a bush, being assembled around the pin and made of a low-friction and highly wearproof material, is used between the two connected ends of the joint.

Although advantageous under different points of view and responding to the aim of allowing angular displacements on a predetermined plane (which is a plane being substantially perpendicular to the pin axis of symmetry), an articulated joint arranged as above schematically described has the side-drawback of preventing any lateral angular displacement with respect to this plane, centred along the pin axis of symmetry, while a predetermined lateral angular displacement must also be allowed for a total and physiological rehabilitation of the articulation.

### Summary of the invention

The problem underlying the present invention is to provide an articulated joint for orthopaedic use, capable of overcoming the drawbacks mentioned with reference to the prior art.

The problem is solved, according to the present invention, by an articulated joint for orthopaedic use, as above described and characterised in that said insert is equipped with a convex surface, having a predetermined bending, facing said fork wing.

Further features and the advantages of the articulated joint for orthopaedic use, according to the present invention, will be apparent from the following description of an embodiment thereof made with reference to the attached drawings, given by way of non-limiting example.

### Brief description of the drawings

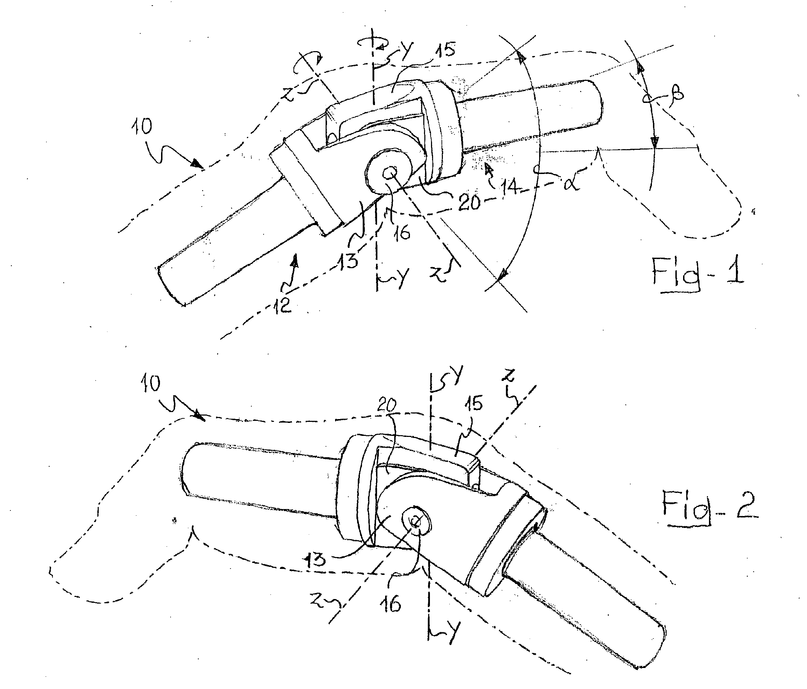

Figure 1 is a schematic perspective view of an articulated joint for orthopaedic use according to the invention.
Figure 2 is a schematic perspective view of the joint of figure 1, from a different lookout point.

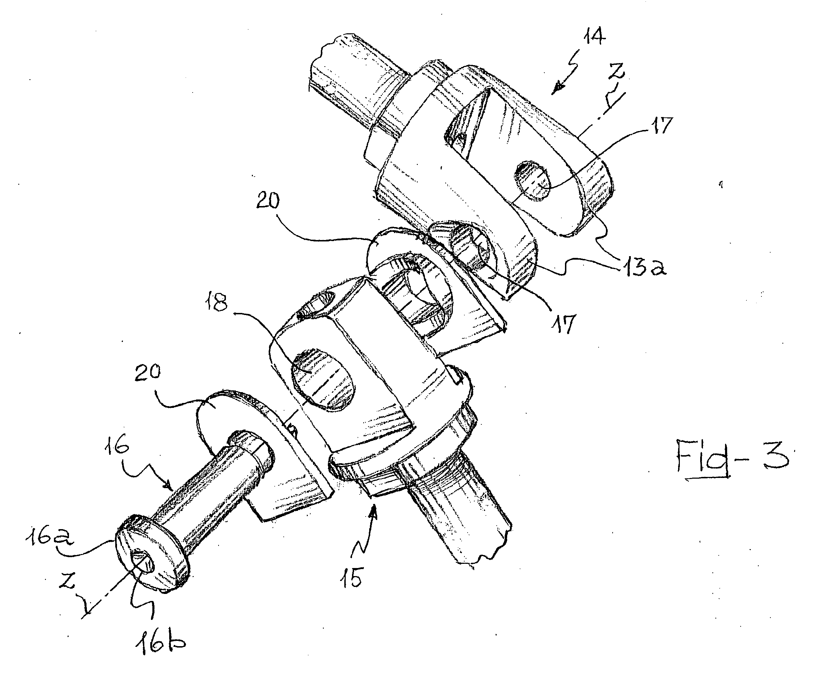

Figure 3 is an exploded perspective view of the joint of figure 1.

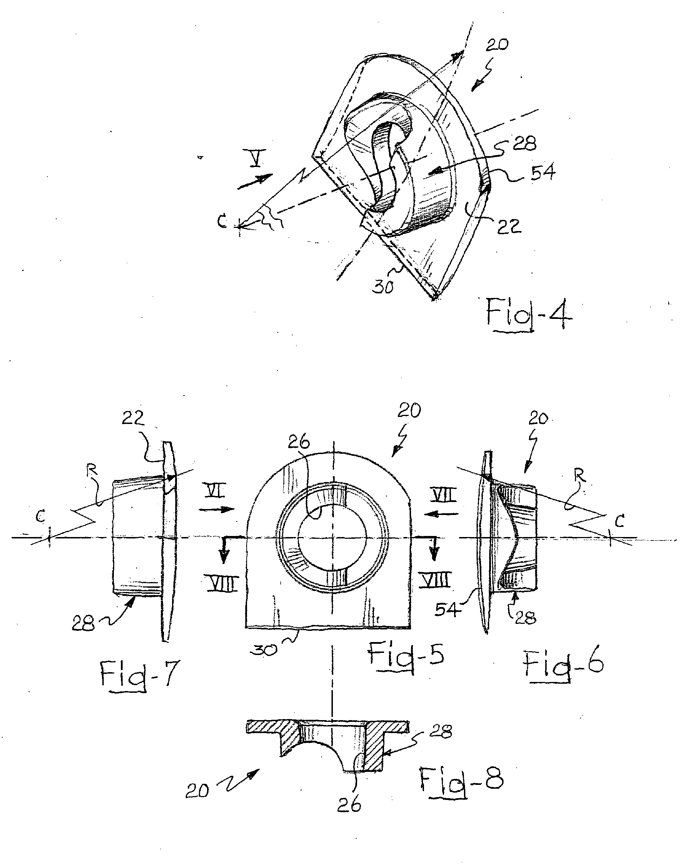

Figure 4 is an enlarged-scale perspective view of a detail of figure 3.
Figure 5 is a plan view of the detail of figure 4.
Figure 6 is a side-elevation view of the detail of figure 4.
Figure 7 is a side-elevation view of the detail of figure 4, taken from an opposite lookout point with respect to that of figure 6.
Figure 8 is a cross section of the detail of figure 5, taken according to the traced VIII-VIII plane of figure 5.

### Detailed description of a preferred embodiment

With reference to the drawings, an articulated joint for orthopaedic use is shown, according to the present invention and globally indicated with 10.

The joint 10 comprises a first half part 12 to be associated for example to a proximal bone, equipped with a fork-shaped end 13 with two wings 13a, and a second half part 14 to be associated for example to a distal bone, equipped with a male end 15 which can be inserted in the fork 13, said ends being hinged to each other by means of a pin 16. Therefore, the proximal bone and the distal bone, connected to the two half parts 12 and 14 respectively, can move angularly, reciprocally, around the pin 16, having axis Z-Z.

It is shown that it is alternatively possible to assemble the first half part 12 on the distal bone and the second half part 14 on the proximal bone, the joint 10 of the invention always being obviously of the same type as described hereafter. In the example of the drawings, the pin 16 connects the fork 13 and the male end 15 passing through respective through holes 17 and 18 of the fork 13 and of the male end 15. In the example shown, each of the two wings 13a of the fork 13 has a respective through hole 17, the two through holes 17 being substantially coaxial.

The pin 16 has a reference head 16a, with a recess 16b for centring the joint 10, and, for example, for positioning in a stable way a tool suitable for inserting the pin 16. An opposite end to the reference head 16a is equipped with a convex surface, suitable for favouring the abutment of a punch for extracting the pin 16.

It must be specified that the diameters of the holes 17 of the two wings 13a of the fork 13 are not identical: a hole 17 allows the pin 16 to be inserted with backlash, while the pin 16 engages in the other hole 17 with interference. The diameter of the hole 18 has a predetermined backlash with respect to the diameter of the pin 16.

At least one ring-shaped insert 20 is interposed between facing walls of the fork 13 and of the male end 15, it is assembled around the pin 16 and it is made of a low-friction and highly wearproof material. This material is for example Peek Optima® or ultra-high-molecular-weight polyethylene UHMWPE or aluminium.

In the example shown, the inserts 20 are two, they are specular and located at two opposite sides of the male end 15, each one being thus located at each wing 13a of the fork 13.

According to an aspect of the present invention, said at least one insert 20 is substantially a holed plate: it has a fundamentally flat surface 22 on the side abutting on the male end 15, while it has a convex surface 24 of predetermined bending on the opposite side, which faces said wing 13a of the fork 13. Preferably, said convex surface 24 is substantially a portion of cylindrical surface, said cylinder having the axis being substantially perpendicular to the axis Z-Z of said pin 16 (in the drawings, the trace of this axis on a perpendicular plane thereof is indicated with C). For example, the bending radius R of said cylindrical surface is comprised in a range of four-ten times the distance between the two wings 13a of the fork 13: in particular, with reference to the non-limiting example of an articular prosthesis reconstructing an articulation between the metacarpus and a phalanx or between two phalanxes of an hand, a mean value generally used for the bending radius R is 4 mm.

The hole 26 of the insert 20 is located at the through hole 18 of the male end 15. More precisely, on the side providing the fundamentally flat surface 22, a substantially cylindrical collar 28, which is sized to be inserted in the through hole 18 of the male end 15, projects around this hole 26: the hole 26 is internally frusto-conical, countersunk towards the portion of convex surface 24.

In the drawings, the collar 28 is shown with a lack of material, for example suitable for allowing a tool to pass in an assembly step of the second half part 14.

It must be specified that stopping means are provided between the two half parts 12 and 14 to define a predetermined maximum angular displacement α of the two half parts 12 and 14. Preferably, the maximum angular displacement α is approximately 90°.

Moreover, the inserts 20 are equipped with means for a predetermined positioning on the male end 15, defining a predetermined orientation of said inserts 20 on the male end 15, preventing a relative rotation between the inserts 20 and the second half part 14. In the drawings, these means for a predetermined positioning comprise a flat abutment surface 30, being orthogonal to the insert fundamentally flat surface 22, corresponding to a similar surface formed on the male end 15.

An alternative embodiment of the present invention is to make the inserts 20 integral with said male end 15 through association means, for example of chemical type (such as convenient adhesives) or of mechanical type (such as respective threads between inserts 20 and male end 15).

A further alternative embodiment of the present invention is to produce said inserts 20 enbloc, integrated with said male end 15.

The operation of the articulated joint for orthopaedic use according to the invention is specified hereafter.

First, connections are created between the two half parts 12 and 14 and the respective proximal and distal bones.

Once the through holes 17 and 18 of the fork 13 and of the male end 15 have been aligned, the pin 16 is passed therethrough, caring for interposing the two inserts 20.

As already mentioned, each insert 20 is located with the substantially cylindrical collar 28 in the through hole 18 of the male end 15, the convex surface 24 facing thus the inner wall of a wing 13a of the fork 13.

In practise, it can be noticed that, by means of the convex surfaces 24, the joint 10 can allow small lateral angular displacements β with respect to the natural rotation plane of the first half part 12 around the second half part 14, the plane being, as mentioned, substantially perpendicular to the axis Z-Z of the pin 16.

These lateral angular displacements β, occurring around an axis Y-Y being substantially perpendicular to the axis Z-Z of the pin 16, are predetermined and, by way of example, they can be of 5° on one side of said plane and of 5° on the other side.

The main advantage achieved by the articulated joint for orthopaedic use according to the present invention is that it allows an unusually complete rehabilitation of the articulation whereon the orthopaedic operation has been performed, predetermined lateral rotations being also provided.

Obviously, to meet specific and contingent needs, a skilled in the art could make several changes to the above-described articulated joint for orthopaedic use, all comprised however in the scope of protection of the present invention, as defined in the following claims.

## Claims

1. Articulated joint (10) for orthopaedic use, comprising a first half part (12), equipped with a fork-shaped end (13) with two wings (13a), and a second half part (14), equipped with a male end (15) which can be inserted in the fork (13), said ends (13, 15) being hinged to each other by means of a pin (16) and thus angularly movable around said pin (16), at least one ring-shaped insert (20) being interposed between facing walls of said fork (13) and of said male end (15), said at least one insert (20) being assembled around said pin (16), **characterised in that** said insert (20) is equipped with a convex surface (24), of predetermined bending, facing said wing (13a) of the fork (13).

2. Articulated joint (10) according to claim 1, **characterised in that** said convex surface (24) is substantially a portion of cylindrical surface, said cylinder having an axis being substantially perpendicular to the axis (Z-Z) of said pin (16).

3. Articulated joint (10) according to claim 2, **characterised in that** the bending radius (R) of said cylindrical surface is comprised in a range of four-ten times the distance between the two wings (13a) of the fork (13).

4. Articulated joint (10) according to claim 1, **characterised in that** said pin (16) connects said fork (13) and said male end (15) passing through respective through holes (17, 18) of said fork (13) and of said male end (15).

5. Articulated joint (10) according to claim 1 or 4, **characterised in that** each of said two wings (13a) of the fork (13) has a respective through hole (17), the two through holes (17) being substantially coaxial.

6. Articulated joint (10) according to claim 1, **characterised in that** said pin (16) has a reference head (16a), with a recess (16b) for centring said joint 10.

7. Articulated joint (10) according to claim 6, **characterised in that** an opposite end to said reference head (16a) is equipped with a convex surface, suitable for favouring the abutment of a punch for extracting said pin (16).

8. Articulated joint (10) according to claim 5, **characterised in that** the diameters of said holes (17) of the two wings (13a) of the fork (13) are different, an hole (17) allowing said pin (16) to be inserted with backlash, while said pin (16) engaging in the other hole (17) with interference.

9. Articulated joint (10) according to claim 4 or 8, **characterised in that** the diameter of a hole (18) of said fork (13) has a predetermined backlash with respect to the diameter of said pin (16).

10. Articulated joint (10) according to claim 1, **characterised in that** said insert (20) is made of a low-friction and highly wearproof material.

11. Articulated joint (10) according to claim 10, **characterised in that** said material is Peek Optima®.

12. Articulated joint (10) according to claim 10, **characterised in that** said material is ultra-high-molecular-weight polyethylene UHMWPE .

13. Articulated joint (10) according to claim 10, **characterised in that** said material is aluminium.

14. Articulated joint (10) according to claim 1, **characterised in that** said inserts (20) are two, they are specular and located at two opposite sides of said male end (15), each one being thus located at each wing (13a) of the fork (13).

15. Articulated joint (10) according to claim 1, **characterised in that** said insert (20) is substantially a holed plate, having a fundamentally flat surface (22) on the side abutting on the male end (15), while it has said convex surface (24) on the opposite side.

16. Articulated joint (10) according to claim 15, the hole (26) of the holed plate is located at the through hole (18) of the male end (15).

17. Articulated joint (10) according to claim 16, **characterised in that** on the side providing said fundamentally flat surface (22) a substantially cylindrical collar (28), which is sized to be inserted in the through hole (18) of the male end (15), projects around said hole (26).

18. Articulated joint (10) according to claim 17, **characterised in that** said hole (26) of the holed plate is internally frusto-conical, countersunk towards said convex surface (24).

19. Articulated joint (10) according to claim 1, **characterised in that** stopping means are provided between the two half parts (12, 14) to define a predetermined maximum angular displacement (α) of said two half parts (12, 14).

20. Articulated joint (10) according to claim 19, **characterised in that** said maximum angular displacement (α) is approximately 90°.

21. Articulated joint (10) according to claim 1, **characterised in that** said insert (20) are equipped with means for a predetermined positioning on the male end (15), defining a predetermined orientation of said insert (20) on the second half part (14).

22. Articulated joint (10) according to claims 15 and 21, **characterised in that** said means for a predetermined positioning comprise a flat abutment surface (30), being orthogonal to the fundamentally flat surface (22) of the insert (20), corresponding to a similar surface formed on the male end (15).

23. Articulated joint (10) according to claim 1, **characterised in that** said insert (20) is made integral with said male end (15) through association means.

24. Articulated joint (10) according to claim 23, **characterised in that** said association means are of chemical type.

25. Articulated joint (10) according to claim 23, **characterised in that** said association means are of mechanical type.

26. Articulated joint (10) according to claim 1, **characterised in that** said insert (20) and said male end (15) are integrated enbloc.
